Europäisches Patentamt

European Patent Office

Office européen des brevets

(11) Veröffentlichungsnummer: **0 331 782**
**A1**

(12) ## EUROPÄISCHE PATENTANMELDUNG

(21) Anmeldenummer: **88103741.0**

(22) Anmeldetag: **09.03.88**

(51) Int. Cl.⁴: **A61H 33/04 , A61N 5/06**

(43) Veröffentlichungstag der Anmeldung:
**13.09.89 Patentblatt 89/37**

(84) Benannte Vertragsstaaten:
**AT BE CH DE ES FR GB IT LI NL**

(71) Anmelder: **PSORI-MED AG**
**Moosmattstrasse 15**
**CH-6330 Cham(CH)**

(72) Erfinder: **Schydlo, Martin T.**
**Am Obersthof 25**
**D-4030 Ratingen(DE)**

(74) Vertreter: **Liesegang, Roland, Dr.-Ing. et al**
**FORRESTER & BOEHMERT**
**Widenmayerstrasse 4 Postfach 22 01 37**
**D-8000 München 22(DE)**

(54) **Duschvorrichtung.**

(57) Eine Duschvorrichtung zum Duschen mit einer Behandlungsflüssigkeit, wie einer Sole, weist eine Duschkabine (21) mit einem Behandlungsraum (20) auf, in der eine Verspritzeinrichtung (6) zum Spritzen der Behandlungsflüssigkeit sowie eine Bestrahlungseinrichtung (7) zur Abgabe von UV-Strahlung an den Körper der duschenden Person auf.

Fig. 1

EP 0 331 782 A1

## Duschvorrichtung

Die Erfindung betrifft eine Duschvorrichtung zum Duschen mit einer Behandlungsflüssigkeit, wie einer Sole, insbesondere zur Behandlung von Hautkrankheiten, wie Psoriasis, Neuro-Dermitis oder dgl.

Es ist bekannt, Hautkrankheiten, wie Psoriasis, durch Baden in einer Sole-Lösung enthaltend eine Salzkonzentration, wie sie ähnlich im Toten Meer in der Natur vorkommt, kombiniert mit einer UV-Bestrahlung zu behandeln.

Eine Salzmischung zur Behandlung der Psoriasis ist in EP-A-0 217 975 beschrieben. Eine Solebadvorrichtung ist in EP-A-0 221 199 beschrieben. Eine solche Badvorrichtung ist aufwendig und in der Praxis für die Behandlung von mehreren Patienten gleichzeitig in Spezialkliniken konzipiert.

Der Erfindung liegt die Aufgabe zugrunde, eine Duschvorrichtung für die Behandlung von Hautkrankheiten, insbesonder Psoriasis, Neuro-Dermitis oder dgl., anzugeben, die zur Anwendung in jeder Hautarzt-Praxis geeignet oder sogar beim Patienten zu Hause mit wenig Aufwand installierbar und betreibbar ist.

Zur Lösung dieser Aufgabe dienen die Merkmale des Anspruchs 1.

Bei einer ersten Ausführung der Duschvorrichtung nach der Erfindung ist ein Behälter für den in das Leitungswasser einzumischenden Behandlungsstoff sowie eine Pumpe zum Umwälzen der Behandlungsflüssigkeit im Kreislauf aus einem Duschbecken zur Verspritzeinrichtung auf den Körper der Person und zurück in das Duschbecken vorgesehen. Dabei kann der Behälter eine Dosiervorrichtung zum Einmischen des Behandlungsstoffes in des Leitungswasser aufweisen.

Bei einer anderen Ausführung der Erfindung ist ein Behälter für die Behandlungsflüssigkeit aus Leitungswasser und dem Behandlungsstoff in einem Kreislauf enthaltend ein Duschbecken, eine Pumpe und die Verspritzeinrichtung einschaltbar. In diesem Fall wird beispielsweise die Mischung aus dem Behandlungsstoff und dem Leitungswasser im Duschbecken hergestellt und dann über die Pumpe der Verspritzeinrichtung und von dort an den Körper des Patienten gebracht, um anschließend wieder im Duschbecken gesammelt und für die nächste Behandlung in dem Behälter gespeichert zu werden.

Bei beiden Ausführungen ist vorteilhaft, wenn das Duschbecken zur Aufnahme der für eine Behandlung erforderlichen Leitungswassermenge bemessen ist und einen Ablauf mit zwei wahlweise einzustellenden Stellungen hat, in deren einer der Ablauf mit einem Abfluß und in deren anderer der Ablauf mit dem Kreislauf verbunden ist.

Weitere Ausgestaltungen der Erfindung sind in den Unteransprüchen 5 bis 12 angegeben. Dabei erlaubt die Ausgestaltung nach Anspruch 12 der Duschvorrichtung nach der Erfindung auch die Einführung von Dampf in den Behandlungsraum, was nicht nur zur Behandlung von Hautkrankheiten sondern auch zur Behandlung der Erkrankung der Atemwege eines Patienten ausnützbar ist.

Die Duschvorrichtung nach der Erfindung ermöglicht ganz allgemein das Duschen mit einem Gemisch aus Leitungswasser und einem nach Wahl des Duschenden dosiert einmischbaren Behandlungsstoff einmal oder mehrfach. Dabei kann die im Kreislauf umzuwälzende Menge an Behandlungsflüssigkeit vergleichsweise klein (zwischen 5 und 15 l) gehalten werden, was sehr niedrige, auch von einem einzelnen Patienten aufbringbare Behandlungskosten zur Folge hat.

Die Duschvorrichtung nach der Erfindung ist nicht auf die Behandlung von Krankheiten der erwähnten Art beschränkt sondern kann auch für andere, z.B. kosmetische Zwecke, eingesetzt werden.

Die Erfindung ist im folgenden anhand schematischer Zeichnungen an einem Ausführungsbeispiel mit weiteren Einzelheiten näher erläutert. Es zeigen:

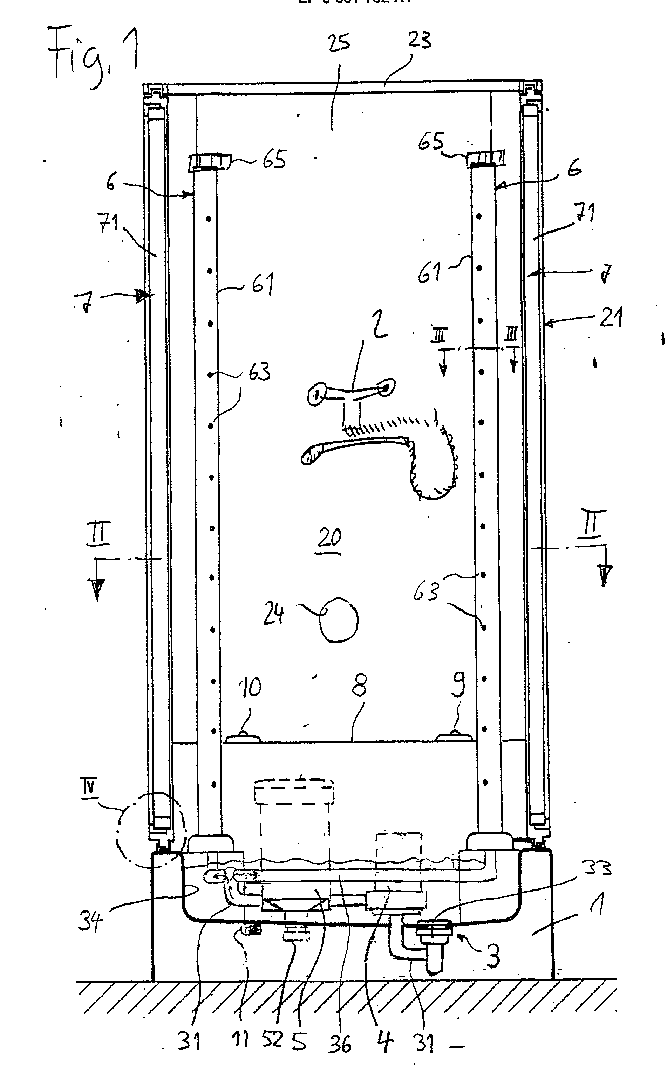

Fig. 1 eine teilweise geschnittene Seitenansicht einer Duschvorrichtung gemäß der Erfindung mit geöffneter Duschkabine;

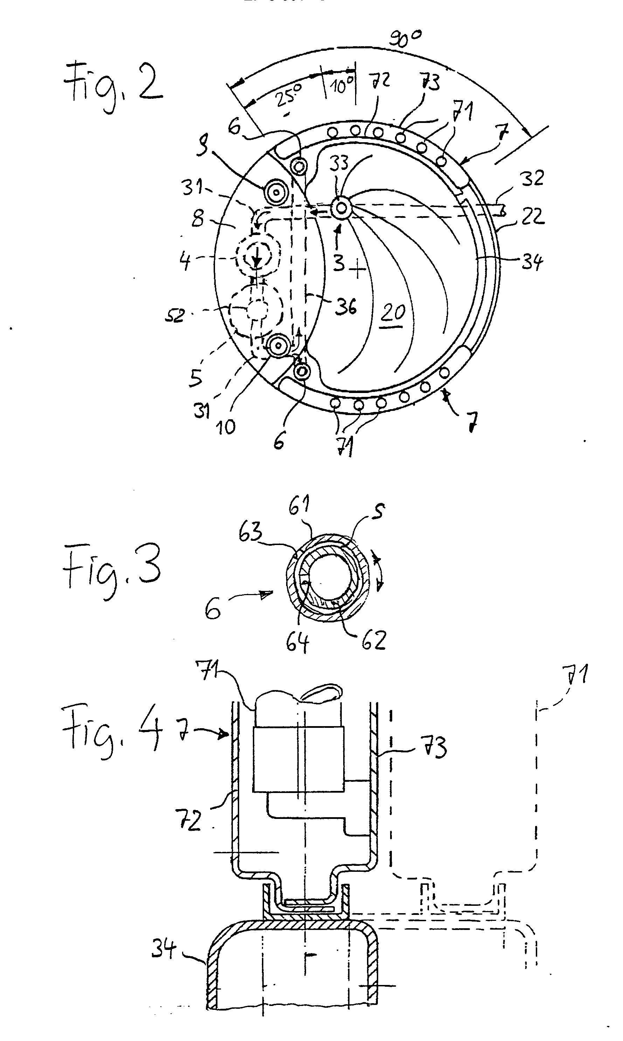

Fig. 2 einen Schnitt nach der Linie II-II in kleinerem Maßstab;

Fig. 3 einen Querschnitt in größerem Maßstab nach der Linie III-III in Fig. 1 durch eine Verspritzeinrichtung gemäß der Erfindung;

Fig. 4 eine Einzelheit bei IV in Fig. 1 im Schnitt.

Die gezeigte Duschvorrichtung weist eine runde Duschkabine 21 mit Tür 22, abnehmbaren Dach 23 und Rückwand 25 auf. Die Duschkabine 21 umgrenzt einen Behandlungsraum 20. In der Rückwand ist eine übliche Duscharmatur 2 für Leitungswasser installiert. Ferner ist die Rückwand von einem Dampfeinlaß 24 durchsetzt.

Die Duschkabine 21 ist auf ein Duschbecken 34 mit Ablauf 3 aufgesetzt. An der Rückwand 25 ist ein Sitz mit abnehmbarer Sitzfläche 8 angeformt. Unter dem Sitz ist ein Behälter für einen Behandlungsstoff, wie ein Salzgemisch mit einer Zusammensetzung gleich oder ähnlich den natürlich im Toten Meer vorkommenden Salzen vorgesehen. Dieser Behälter 5 weist eine Dosiervorrichtung zur dosierten Entnahme von Salz in Form z. B. eines von Handrades 52 bzw. einer auf eine vorwählbare Ausbringmenge je Zeiteinheit einstellbare automatische Vorrichtung zum kontinuierlichen Ausbringen von Salz auf und ist in einer Bypassleitung 31 angeordnet, deren einer Zweig über eine Umwälzpumpe 4 mit dem Ablauf 3 verbunden ist, während ihr anderer Zweig mit einer Verbindungsleitung 36 zwischen zwei vertikal und jeweils an einer Seite des Sitzes 8 angeordneten Verspritzeinrichtungen 6 verbunden ist. Die Verspritzeinrichtungen 6 umfassen gemäß Fig. 3 jeweils zwei koaxial mit einem Spiel s von ca. 0,2 mm ineinandersteckenden Rohren, nämlich einem Innenrohr 61 und einem Außenrohr 62 mit jeweils in Längsreihen angeordneten Spritzlöchern 63 bzw. 64. Die Rohre 61, 62 sind relativ zueinander über je ein Handrad 65 verdrehbar, um mögliche Salzverkrustungen zu beseitigen. Beidseitig im Bereich zwischen der Tür 22 und dem Sitz 8 sind jeweils zwei Gruppen von Bestrahlungseinrichtungen 7 mit jeweils sechs UV-Röhren in einem Gehäuse angeordnet, das behandlungsraumseitig eine transparente Wand 72 und außenseitig eine Montagewand 73 für die Befestigung und Stromversorgung der Röhren 71 aufweist.

In der Sitzfläche 8 sind ein Schalter 9 zum Betätigen der Pumpe 4 sowie ein Schalter 10 zum Ein- und Ausschalten der Bestrahlungseinrichtungen 7 installiert.

Auf der Unterseite des Duschbeckens 34 ist ein induktiver Schalter 11 angeordnet, der auf das Füllen des Duschbeckens 34 mit Wasser anspricht und in diesem Fall die elektrische Versorgung für die Pumpe 4 und die Bestrahlungseinrichtungen 7 aktiviert.

Der Ablauf 3 enthält einen Stöpsel 33 mit zwei Stellungen, in deren einer er eine Verbindung zwischen Duschbecken 34 und einer Abflußleitung 32 freigibt, während er in der anderen Stellung eine Verbindung zwischen dem Duschbecken 34 und der Leitung 31 freigibt.

Mit der beschriebenen Duschvorrichtung duscht man wie folgt:

Das Duschbecken 34 wird nach Füllen über die Armatur 2 mit Leitungswasser über die Leitung 31 mit der Pumpe 4 verbunden. Aus dem Behälter 5 kann Salz in einer zweckentsprechenden Menge (z.B. ca. 1 1/2 kg Salz auf 8 l Leitungswaser) vorher entnommen und in das Duschbecken 34 eingestreut worden sein. Es ist aber auch möglich, in das durch die Pumpe 4 bewegte Leitungswasser automatisch eine proportionale Salzmenge aus dem Behälter 5 in das Leitungswasser einzumischen. Die so gebildete Sole wird von der Pumpe 4 über den stromabwärts vom Behälter 5 gelegenen Leitungszweig der Bypassleitung 31 in die Verbindungsleitung 36 und von dort zu den beiden Verspritzeinrichtungen 6 geführt. Durch Verdrehen der Spritzrohre zueinander kann die Spritzwirkung stärker oder schwächer gestellt werden. Nach dem Verspritzen der Behandlungsflüssigkeit auf den Körper des Duschenden gelangt die Flüssigkeit zurück in das Duschbecken 34, und der Kreislauf kann bei weiter gesperrter Verbindung des Ablaufes 3 mit dem Abflußrohr 32 beliebig wiederholt werden.

Zu Beginn des Duschvorganges hat der Duschende mittels des Schalters 10 die Bestrahlungseinrichtungen 7 eingeschaltet, so daß sein Körper gleichzeitig mit dem Duschen mit der Behandlungsflüssigkeit einer UV-Bestrahlung ausgesetzt wird.

Der Duschende kann während des Duschvorganges sowohl stehen als auch sitzen.

Soll mit der Duschvorrichtung zusätzlich eine Dampfbehandlung ermöglicht werden, beispielsweise zum Inhalieren, wird ein nicht gezeigter Dampfgenerator, der drucklos arbeitet, von außen her mit dem Dampfeinlaß 24 gekuppelt.

Nach dem Duschen mit der Behandlungsflüssigkeit kann mittels der Armatur 2 mit normalem Leitungswasser abgeduscht werden. Hierzu wird der Stöpsel 33 in eine Stellung gebracht, in der das Duschbecken 34 mit der Abflußleitung 32 verbunden ist.

## Ansprüche

1. Duschvorrichtung zum Duschen mit einer Behandlungsflüssigkeit, wie einer Sole, insbesondere zur Behandlung von Hautkrankheiten, wie Psoriasis, Neuro-Dermitis oder dgl., dadurch **gekennzeichnet** daß in einer Duschkabine (21) mit einem Behandlungsraum (20) für eine Person eine Verspritzeinrichtung (6) zum Spritzen einer Behandlungsflüssigkeit aus Leitungswasser und Behandlungsstoff, insbesondere ein Salzgemisch, sowie eine Bestrahlungseinrichtung (7) zur Abgabe von UV-Strahlung an den Körper der Person angeordnet sind.

2. Duschvorrichtung nach Anspruch 1, **gekennzeichnet** durch einen Behälter (5) für den in Leitungswasser einzumischenden Behandlungsstoff sowie eine Pumpe (4) zum Umwälzen der Behandlungsflüssigkeit im Kreislauf aus einem Duschbecken (34) zur Verspritzeinrichtung (6) auf den Körper der Person und zurück in das Duschbecken (34).

3. Duschvorrichtung nach Anspruch 1, **gekennzeichnet** durch einen Behälter (5) für die Behandlungsflüssigkeit aus Leitungswasser und dem Behandlungsstoff, der in einen Kreilauf enthaltend ein Duschbecken (34), eine Pumpe (4) und die Verspritzeinrichtung (6) einschaltbar ist.

4. Duschvorrichtung nach Anspruch 2 oder 3, dadurch **gekennzeichnet**, daß das Duschbecken (34) zur Aufnahme der für eine Behandlung erforderlichen Leitungswassermenge bemessen ist und einen Ablauf (3) mit zwei wahlweise einzustellenden Stellungen hat, in deren einer der Ablauf mit einem Abfluß (32) und in deren anderer der Ablauf mit dem Kreislauf verbunden ist.

5. Duschvorrichtung nach Anspruch 2 oder 4, dadurch **gekennzeichnet**, daß an dem Behälter (5) eine Dosiervorrichtung (52) zum Einmischen des Behandlungsstoffes in das Leitungswasser angeordnet ist.

6. Duschvorrichtung nach einem der Ansprüche 1 bis 5, dadurch **gekennzeichnet**, daß die Verspritzeinrichtung (6) mindestens ein vertikal in der Duschkabine (21) seitlich im Behandlungsraum (20) angeordnetes Spritzrohr (61) mit über die Länge verteilten Spritzlöchern (63) aufweist.

7. Duschvorrichtung nach Anspruch 6, dadurch **gekennzeichnet**, daß das oder jedes spritzrohr ein Außenrohr (61) und ein relativ dazu bewegbares Innenrohr (62) mit Spritzlöchern (63,64) aufweist, um Verkrustungen des Behandlungsstoffes beseitigen zu können.

8. Duschvorrichtung nach einem der Ansprüche 1 bis 7, dadurch **gekennzeichnet**, daß mehrere UV-Röhren (71) vertikal um den Behandlungsraum (20) herum zu dessen gleichmäßiger Ausleuchtung vorzugsweise in zwei Gruppen angeordnet sind.

9. Duschvorrichtung nach einem der Ansprüche 1 bis 8, dadurch **gekennzeichnet**, daß Schalter (9,10) zur Betätigung der Pumpe (4) und der Bestrahlungsvorrichtung (7) an für die Person leicht zugänglichem Ort (Sitzfläche 8) im Behandlungsraum (20) angeordnet sind.

10. Duschvorrichtung nach einem der Ansprüche 1 bis 9, dadurch **gekennzeichnet**, daß dem Boden des Duschbeckens ein induktiver Sensor (11) zum Aktivieren der Pumpe (4), der Bestrahlungsvorrichtung (7) und der Betätigungsvorrichtung (9,10) zugeordnet ist.

11. Duschvorrichtung nach einem der Ansprüche 1 bis 10, dadurch **gekennzeichnet**, daß zusätzlich zu der Verspritzeinrichtung (6) eine Leitungswasserdusche (2) vorgesehen ist.

12. Duschvorrichtung nach einem der Ansprüche 1 bis 11, dadurch **gekennzeichnet**, daß die Duschkabine (21) ein Dach (23), eine mittels einer Tür (22) oder dgl. dicht abschließbare Zugangsöffnung zum Behandlungsraum (20) sowie einen Dampfeinlaß (24) aufweist.

Fig. 1

EP 0 331 782 A1

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | NL-A-8 503 423  (VELDBOER) <br> * Figuren 1-4; Seite 2, Zeile 30 - Seite 3, Zeile 34 * | 1-3,5,7 ,11 | A 61 H  33/04 <br> A 61 N   5/06 |
| Y | | 6,9-10, 12 | |
| Y | US-A-3 458 874  (FRITZ) <br> * Figuren 1-3; Spalte 2, Zeilen 6-8,24-29 * | 6 | |
| Y | EP-A-0 122 705  (AMERICAN STANDARD INC.) <br> * Figur 1; Seite 5, Zeilen 14-16; Seite 6, Zeilen 19-23 * | 9-10 | |
| Y | US-A-3 885 557  (VAREA) <br> * Figuren 1-3; Zusammenfassung; Spalte 2, Zeile 62 - Spalte 3, Zeile 2 * | 12 | |
| A | | 9 | |
| X | DE-A-3 322 941  (LASKOWSKI & SCHLICH) <br> * Figuren 1-2; Seite 5, Absätze 3,5; Seite 8, Absatz 2 * | 1,8 | **RECHERCHIERTE SACHGEBIETE (Int. Cl.4)** <br><br> A 61 H <br> A 61 N |
| A | DE-A-3 317 140  (BEHLENDORF) <br> * Figuren 1,2; Seite 4, letzter Absatz - Seite 5, Absatz 4 * | 8 | |

Der vorliegende Recherchenbericht wurde für alle Patentansprüche erstellt

| Recherchenort | Abschlußdatum der Recherche | Prüfer |
|---|---|---|
| DEN HAAG | 20-10-1988 | JONES T.M. |